Europäisches Patentamt

European Patent Office

Office européen des brevets

(19)

(11) **EP 1 256 354 A1**

## (12) EUROPEAN PATENT APPLICATION

(43) Date of publication:
**13.11.2002 Bulletin 2002/46**

(51) Int Cl.[7]: **A61K 47/48**, C07K 14/52

(21) Application number: **01401211.6**

(22) Date of filing: **11.05.2001**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **SCHERING CORPORATION
Kenilworth New Jersey 07033 (US)**

(72) Inventors:
- **Vicari, Alain P.
69890 La Tour de Salvagny (FR)**
- **Caux, Christophe
01360 Bressolles (FR)**

(74) Representative: **UEXKÜLL & STOLBERG
Patentanwälte
Beselerstrasse 4
22607 Hamburg (DE)**

(54) **Methods for treating cancer**

(57) Dendritic cells play a critical role in antigen-specific immune responses. Materials and methods are provided for treating disease states, including cancer and autoimmune disease, by facilitating the migration or activation of antigen-presenting dendritic cells. In particular, methods are provided for treating cancer in a mammal comprising administering to said mammal an effective amount of a targeting construct comprising 6Ckine or a biologically active fragment or variant thereof and a targeting moiety.

**Description**

The invention relates to the use of chemokines in the treatment of disease states, especially cancer.

**Background of the invention**

Dendritic cells (DC) specialize in the uptake of antigen and their presentation to T cells. DC thus play a critical role in antigen-specific immune responses. (Caux, et al., 1995, Immunology Today 16:2; Steinman, 1991, Ann. Rev. Immunol. 9:271-296). This process involves the capture and processing of antigens by DC in the periphery, their migration to regional lymph nodes via the lymphatics and the presentation of the processed antigens to T cells. (Banchereau et al, 1998, Nature 392: 6673:245).

In recent years, investigators have attempted to exploit the activity of DC in the treatment of cancer. In an animal model, as few as $2 \times 10^5$ antigen-pulsed DC will induce immunity when injected into naive mice (Inaba at al., 1990, Intern. Rev. Immunol. 6:197-206). Flamand et al. (Eur. J. Immunol., 1994, 24:605-610) pulsed mouse DC with the idiotype antigen from a B-cell lymphoma and injected them into naive mice. This treatment effectively protected the recipient mice from subsequent tumor challenges and established a state of lasting immunity. Injection of antigen alone, or B cells pulsed with antigen, had no effect, suggesting that it was the unique characteristics of DC that were responsible for the anti-tumor response. It has been postulated that DC are not only capable of inducing anti-tumor immunity, but that they are absolutely essential for this process to occur (Ostrand-Rosenberg, 1994, Current Opinion in Immunol. 6:722-727; Grabbe et al., 1995, Immunol. Today 16:117-120; Huang et al., 1994, Science 264: 961-965). Huang and coworkers (Huang et al., 1994, Science 264:961-965) inoculated mice with a B7-1 transfected tumor that was known to produce anti-tumor immunity. They demonstrated that only mice with MHC-compatible APC were capable of rejecting a tumor challenge. In mice, tumor antigen-loaded in vitro generated DC have been shown, by various groups, to prevent the development of tumors and more importantly to induce the regression of established tumors ( Mayordomo et al., 1995, Nat. Med. 1:1297-1302; Paglia et al., 1996, J. Exp. Med. 193:317-322).

Studies in humans have demonstrated a similar role for DC. It has been reported that peptide-specific CTL are readily induced from purified CD8+ T cells using peptide-pulsed DC, but are not elicited when peptide-pulsed monocytes are used (Mehta-Damani et al., 1994, J. Immunology 153:996-1003). A patient with advanced B-cell lymphoma was recently treated with DC pulsed with the patient's own tumor idiotype (Hsu et al., 1996, Nature Medicine 2:52). This produced a measurable reduction in the patient's B-cell lymphoma. Treatment of prostate cancer using DC pulsed with PSM antigen has been reported by Murphy et al. (The Prostate 1996 29: 371). A clinical trial has been conducted in which patients with melanoma are being treated with GM-CSF-activated APC pulsed with a peptide from the MAGE-1 tumor antigen (Mehta-Damani et al., 1994, J. Immunology 153:996-1003). Pre-immunization, tumor-infiltrating lymphocytes from two patients were predominantly CD4+ and lacked specific tumor reactivity. In contrast, after immunization, tumor infiltrating lymphocytes from the same patients were predominantly CD8+ and demonstrated MAGE-1 specific anti-tumor cytotoxicity. It thus appears from these studies that DC have a unique and potent capacity to stimulate immune responses. Other phase I clinical trials evaluating the use of DC in cancer have been reviewed in Dallal et al., 2000, Curr. Opin. Immunol. 12: 583-588).

Of significant clinical interest, the histologic infiltration of dendritic cells into primary tumor lesions has been associated with significantly prolonged patient survival and a reduced incidence of metastatic disease in patients with bladder, lung, esophageal, gastric and nasopharygeal carcinoma. In contrast, a comparatively poorer clinical prognosis is observed for patients with lesions that exhibit a sparse infiltration with DC and metastatic lesions are frequently deficient in DC infiltration (Becker, 1993, In Vivo 7:187; Zeid et al., 1993, Pathology 25:338; Furihaton et al., 1992, 61:409; Tsujitani et al., 1990, Cancer 66:2012; Gianni et al., 1991, Pathol. Res. Pract. 187:496; Murphy et al., 1993, J. Inv. Dermatol. 100:3358).

Chemokines are small molecular weight proteins that regulate leukocyte migration and activation (Oppenheim, 1993, Adv. Exp. Med. Biol. 351:183-186; Schall, et al., 1994, Curr. Opin. Immunol. 6:865-873; Rollins, 1997, Blood 90:909-928; Baggiolini, et al., 1994, Adv. Immunol. 55: 97-179). Several chemokines have been shown to attract DC in vitro (Dieu-Nosjean et al., 1999, J. Leukoc. Biol. 66: 252-262; Sozzani, et al., 1995, J. Immunol. 155: 3292-3295; Sozzani, et al., 1997, J. Immunol. 159: 1993-2000; Xu, et al., 1996, J. Leukoc. Biol. 60:365-371; MacPherson, et al., 1995, J. Immunol. 154: 1317-1322; Roake, et al., 1995, J. Exp. Med. 181: 2237-2247). Chemokines are secreted by activated leukocytes themselves, and by stromal cells including endothelial cells and epithelial cells upon inflammatory stimuli (Oppenheim, 1993, Adv. Exp. Med. Biol. 351: 183-186; Schall, et al., 1994, Curr. Opin. Immunol. 6: 865-873; Rollins, 1997, Blood 90: 909-928; Baggiolini, et al., 1994, Adv. Immunol. 55: 97-179). Responses to chemokines are mediated by seven transmembrane spanning G-protein-coupled receptors (Rollins, 1997, Blood 90:909-928; Premack, et al., 1996, Nat. Med. 2: 1174-1178; Murphy, P.M. 1994, Ann. Rev. Immunol. 12: 593-633).

6Ckine belongs to the CC family of chemokines (Hedrick et. al, 1997, J. Immunol. 159:1589-1593). It is also known as CK-beta-9, exodus-2, TCA-4 and SLC (Swiss-Prot accession number 000585 for human protein) and was renamed CCL21 in the new chemokine nomenclature (Zlotnik et al., 1999, J. Immunol. 162: 3765-3769). Human 6Ckine (h6Ckine) binds to the chemokine receptor CCR7, while mouse 6Ckine (m6Ckine) binds to CCR7 as well as to the CXCR3 receptor, although with a lower affinity (Jenh et al., 1999, J. Immunol. 162:37655-36769). The CCR7 receptor is expressed by subsets of dendritic cells (Dieu et al., 1998, J. Exp. Med. 188: 373-386), T and B cells (Nagira et al., 1998, Eur. J. Immunol. 28: 1516-1523; Sallusto et al., 1999, Nature 401: 708-712) and Natural Killer cells (Kim et al., 1999, Cell. Immunol. 193:226-235); 6Ckine was shown to be a chemotactic factor for these cells. In addition, other chemokines binding the CXCR3 receptor such as IP-10 and Mig (Wang et al., 1998, J. Immunol. Methods 220: 1-17) possess angiostatic activity, and such activity has been demonstrated for mouse 6Ckine (Soto et al., 1998, Proc. Natl. Acad. Sci. USA 95: 8205-8210).

Recently, mouse 6Ckine has been shown to have anti-tumor effect when injected into tumors in mice (Sharma et al., 200, J. Immunol. 164:4558-4563). In addition, Vicari et al have shown that C26 colon carcinoma tumor cells engineered to express m6Ckine are less tumorigenic than the parental cell line and that this effect depends on CD8+ cells and Natural killer cell activity in vivo (Vicari et al. 2000, J. Immunol. 165:1992-2000). C26 tumors expressing m6Ckine were found in this study to be significantly infiltrated by dendritic cells and CD8+T cells compared with parental tumors.

The currently available methods of cancer therapy such as surgical therapy, radiotherapy, chemotherapy, and immunobiological methods have either been of limited success or have given rise to serious and undesirable side effects. In many clinically diagnosed solid tumors (in which the tumor is a localized growth), surgical removal is considered the prime means of treatment. However, many times after surgery and after some delay period, the original tumor is observed to have metastasized so that secondary sites of cancer invasion have spread throughout the body and the patient subsequently dies of the secondary cancer growth. Although chemotherapy is widely used in the treatment of cancer, it is a systemic treatment based usually on the prevention of cell proliferation. Accordingly, chemotherapy is a non-specific treatment modality affecting all proliferating cells, including normal cells, leading to undesirable and often serious side effects.

Thus, a need exists for new methods for treating diseases thought to result from aberrant immune responses, especially cancer. Methods and therapies for the modulation of the immune response through the manipulation of dendritic cells will be useful in the treatment of these diseases.

## Summary of the Invention

The present invention fulfills the foregoing need by providing materials and methods for immunotherapy for diseases such as cancer by facilitating the migration and/or activation of antigen-presenting dendritic cells. It has now been discovered that the human chemokine 6Ckine is a useful therapeutic agent for the treatment of cancer. Specifically, human 6Ckine has been found to inhibit tumor growth and metastases. The invention thus provides a method of immunotherapy comprising targeted administration of an effective amount of 6Ckine to a desired site of antigen expression, for example a tumor.

Specifically, the invention provides a method of treating cancer in a mammal comprising administering to said mammal an effective amount of a targeting construct, which targeting construct comprises 6Ckine or a biologically active fragment or variant thereof and a targeting moiety.

In certain embodiment of the invention, the targeting moiety is a peptide, a protein or a small molecule. In other preferred embodiments, the targeting moiety is a vector such as a viral vector. In more preferred embodiments, the targeting moiety is an antibody or antibody fragment. In the most preferred embodiment, the targeting moiety is an ScFv fragment.

In preferred embodiments, the targeting moiety recognizes a tumor associated antigen selected from the group consisting of the folate receptor, Her2/neu receptor, Epidermal Growth Factor Receptor, CA125 tumor antigen, Melan-A, tyrosinase, p97, $\beta$-HCG, GalNAc, MAGE-1, MAGE-2, MAGE-3, MAGE-4, MAGE-12, MART-1, MUC1, MUC2, MUC3, MUC4, MUC18, CEA, DDC, melanoma antigen gp75, HKer 8, high molecular weight melanoma antigen, K19, Tyr1 and Tyr2, members of the pMel 17 gene family, c-Met, PSA, PSM, $\alpha$-fetoprotein, thyroperoxidase, gp100, insulin-like growth factor receptor (IGF-R), telomerase and p53. In other preferred embodiments, the targeting moiety recognizes an antigen associated with the tumor stroma, such as alpha v integrins, the VEGF receptor and the proteoglycan NG2.

In another embodiment of the invention, the methods further comprise administering a substance which allows for slow release of the targeting construct at a delivery site. In certain embodiments, the targeting constructs are administered intravenously, intratumorally, intradennally, intramuscularly, subcutaneously, or topically.

In another aspect of the invention, cytokines are administered in combination, either before or concurrently, with the 6Ckine targeting construct. In one preferred aspect, the cytokines are GM-CSF and IL-4. Administration of GM-CSF and IL-4 stimulates generation of DC from precursors, thereby increasing the number of DC available to capture and process antigen. In another preferred aspect, FLT-3L, or a fusion protein comprising FLT-3L and G-CSF or FLT-3L and GM-CSF is administered in combination with the 6Ckine targeting constructs.

In yet another aspect of the invention, an activating agent such as TNF-α, IFN-α, RANK-L or agonists of RANK, CD40-L or agonists of CD40 and agonists of the toll-like receptor family of molecules such as CpG oligonucleotides (Hemmi et al., 2000, Nature 408: 740-745) is administered in combination with the 6Ckine targeting constructs.

In still another aspect of the invention, other chemokines are administered in combination, either before, after, or concurrently, with the 6Ckine targeting constructs.

Finally, the invention provides targeting constructs comprising 6Ckine or a biologically active fragment or variant thereof and a targeting moiety, and plasmids comprising said targeting constructs. According to the present invention, these targeting constructs may be used for therapeutic applications.

**Brief Description of the Figures**

Figure 1. Human 6Ckine is a chemotactic factor for all subsets of human dendritic cells in human blood.

Figure 2. C26 colon carcinoma cells engineered to express m6Ckine are less tumorigenic compared to parental tumor cells (p<0.01) by logrank analysis (A and B: C26 + control vs C26-6CK + control). Depleting CD8+ cells (A) or Natural Killer cell (B) activity with specific antibodies in vivo partially reverts the delayed tumorigenicity of the C26-6CK tumor cells, indicating that CD8+ cells and NK cells play a role in delaying tumor growth.

Figure 3. C26 wild-type tumors or C26-6CK tumors expressing m6Ckine are significantly infiltrated by CD8+ T cells and CD11c+MHC class II + dendritic cells (DC) compared to C26 tumors.

Figure 4. C26 colon carcinoma tumor cells engineered to express m6Ckine are less angiogenic than parental C26 tumor cells.

Figure 5. Injection of m6Ckine or h6Ckine slows tumor growth in mice in vivo.

Figure 6. Adenoviral delivery of mouse 6Ckine inhibits tumor growth and spontaneous metastasis in established tumors in vivo.

Figure 7. Schematic representation of ph6CKMOV19 or pmCKMOV19 vectors. Following transcription and splicing between SD and SA sites, the recombinant polypeptides consist of the chemokine m6Ckine or h6Ckine fused to MOV19 ScFv via a 13-amino acid spacer and to Cκ, the constant region of κ immunoglobulins. MOV 19 ScFv antibody fragment recognizes the human folate receptor (FR). The deduced molecular weight of both fusion proteins is 46 KDa.

Figure 8. Western blot analysis of purified h6CkineMOV19 (lane 5) and m6CkineMOV19 ScFv (lane 9) fusion proteins.

Figure 9. Injection of hybridomas secreting fusion proteins consisting of m6Ckine or h6Ckine fused to a single chain antibody fragment that recognizes the tumor antigen FR induces a decrease in tumor growth, in tumors that express FR compared to parental C26 tumor cells.

Figure 10. Injection of a recombinant fusion protein consisting of a single chain antibody that recognizes the tumor antigen FR fused to h6Ckine specifically reduces the growth of tumors that express FR.

**Detailed Description of the Invention**

All references cited herein are incorporated in their entirety by reference.

The inventors have discovered that the targeted administration of 6Ckine or a biologically active fragment or variant thereof results in a decrease in tumor growth. Such targeted administration combines the specificity of a targeting moiety with the immunostimulatory activity of 6Ckine. 6Ckine increases the migration of dendritic cells to the site of antigen delivery through interaction with the CCR7 receptor. Dendritic cells uptake and present tumor-derived antigens to initiate an adaptive immune response as well as secrete factors responsible for an innate immune response. In addition, 6Ckine may promote the recruitment into the tumor of effector cells of the immune response such as T cells or Natural Killer cells through the CCR7 and/or CXCR3 receptors. Finally, 6Ckine may exert local angiostatic activity, thereby interfering with the development of the tumor. Targeted administration of 6Ckine may improve tissue penetration and distribution within the tumor and reduce its immunogenicity. The targeting constructs and methods described herein allow for therapeutic treatment, in particular treatment of tumors at distant site by providing local delivery of 6Ckine in a form and concentration that leads to biological effects while reducing the risk of toxicity related to systemic delivery of 6Ckine or of its targeting moiety.

Thus, the invention provides a new treatment for cancer in a mammal comprising administration to said mammal of an effective amount of a targeting construct comprising 6Ckine or a biologically active fragment or variant thereof and a targeting moiety. The invention also relates to the use of a targeting construct comprising 6Ckine or a biologically active fragment or variant thereof and a targeting moiety for the manufacture of a medicament for treating cancer in a mammal.

An "effective amount" of a targeting construct is an amount sufficient to increase the migration of dendritic cells to the site of antigen delivery through interaction with the CCR7 receptor.

The term "6Ckine" as used herein includes biologically active fragments and variants of 6Ckine. Biologically active fragment or variant means a portion or derivation of the 6Ckine molecule which is sufficient to induce the migration of dendritic cells expressing CCR7 at a migration index of at least 2 over control, and/or stimulate a measurable immune response. A measurable immune response can be assessed as an enhanced antigen specific stimulation, preferably against tumor antigens. This include either of the following: i) antigen-specific immunoglobulin levels in serum, typically known as a B-cell response; ii) production of certain classes of immunoglobulins which require T-cell mediated help for isotype switching; iii) production of certain lymphokines such as IFN-γ, TNF-α, GM-CSF, IL-4 by T lymphocytes in response to antigen stimulation; iv) an antigen-specific cytotoxic response of defined populations of lymphocytes (blood, spleen, lymph nodes, tumor). In addition, a measurable immune response against tumors can be assessed by detecting activities which are not specific of tumor antigens but participate in the body response against tumors (and gathered under the term "innate immunity"), such as Natural Killer cell or polynuclear cell activity, release of certain cytokines such as IL-12 and IFN-α, decrease in the production of inhibitory cytokines such as IL-10 and reduced angiogenesis.

"6Ckine" for use in the invention is a natural protein of the body that is active on a restricted subset of cells, in particular dendritic cells, or on other subsets of leucocytes such as T and Natural Killer cells which all express the CCR7 receptor. The term includes naturally occurring mammalian 6Ckine, and variants and fragments thereof, as defined below. Preferably, the 6Ckine is of human or mouse origin. The human and mouse 6Ckine sequences have been deposited in the Swissprot database under accession numbers 009006 (mouse) and 000585 (human).

The 6Ckine used in practicing the invention may be a recombinant protein with an amino-acid sequence identical to the natural product, or a recombinant protein derived from the natural product but including modifications that changes its pharmacokinetic properties and/or add novel biological properties while keeping its original chemoattractant property.

The term "6Ckine" also includes variants and fragments of the natural product. For the purposes of the invention, 6Ckine polypeptides and variants will have at least 70% sequence identity with the natural product polypeptide. Methods for determining the percent sequence identity are discussed below.

"6Ckine variant" refers to a polypeptide derived from the native protein by deletion or addition of one or more amino acids to the N-terminal and/or C-terminal end of the native protein; deletion or addition of one or more amino acids at one or more sites in the native protein; or substitution of one or more amino acids at one or more sites in the native protein. Such variants include mutants, fragments, allelic variants, homologous orthologs, and fusions of native 6Ckine fragments useful in the methods of the invention may be modified by glycosylation, phosphorylation, substitution of non-natural amino acid analogs and the like.

For the purposes of the invention, 6Ckine variants will have at least 70%, generally at least 75%, 80%, 85%, preferably about 90% to 95% or more, and more preferably about 98% or more sequence identity to the amino acid sequence of the native protein.

A variant of the 6Ckine proteins useful in the methods of the invention may differ from the polypeptides disclosed therein by as few as 1-33 amino acid residues, as few as 10-30, as few as 1-15, as few as 1-10, such as 6-10, as few as 5, as few as 4, 3, 2, or even 1 amino acid residue.

Methods for calculating identity and similarity are known in the art. See, for example, Computer Analysis of Sequence Data, Part 1, Griffin, A.M., and Griffin, H.G., eds, Humana Press, New Jersey, 1994; Sequence Analysis in Molecular Biology, von Heinje, G., Academic Press, 1987; and Sequence Analysis Primer, Gribskov, M., and Devereux, J., eds., M Stockton Press, New York, 1991). In general, to determine the percent identity of two amino acid sequences, the sequences are aligned for optimal comparison purposes. The percent identity between the two sequences is a function of the number of identical positions shared by the sequences (i.e., percent identity = number of identical positions/total number of positions (e.g., overlapping positions) x 100). For example, by a polypeptide having an amino acid sequence at least 95% "identical" to a reference amino acid sequence is intended that the amino acid sequence of the polypeptide is identical to the reference sequence except that the polypeptide sequence may include up to five amino acid alterations per each 100 amino acids of the reference amino acid sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

The determination of percent identity between two sequences can be accomplished using a mathematical algorithm. For the purposes of the invention, the percentage sequence identity between two polypeptide sequences is determined using the BESTFIT computer program (Wisconsin Sequence Analysis Package, Version 8 for Unix, Genetics Computer Group, University Research Park, 575 Science Drive, Madison, Wis. 53711), using the default settings. When using BESTFIT to determine whether a particular sequence is, for instance, 95% identical to a reference sequence according to the present invention, the parameters are set, of course, such that the percentage of identity is calculated over the full length of the reference amino acid sequence and that gaps in homology of up to 5% of the total number of amino acid residues in the reference sequence are allowed.

The 6Ckine variants useful in the methods of the invention may be obtained by amino acid substitutions, deletions, truncations, and insertions. Preferred 6Ckine polypeptide variants have one or more conservative amino acid substitutions. For example, conservative amino acid substitutions may be made at one or more amino acid residues. Preferably, substitutions are made at nonessential amino acid residues.

A "nonessential" amino acid residue is a residue that can be altered from the wild-type sequence of a 6Ckine protein without altering the one of the biological activities, whereas an "essential" amino acid residue is required for a given biological activity.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). See, for example, Bowie et al., 1990, Science 247:1306, herein incorporated by reference. Preferably, such substitutions would not be made for conserved cysteine residues, such as the amino terminal contiguous cysteine residues.

The 6Ckine variants useful in the methods of the invention may be isolated from naturally occurring variants, isolated after mutagenesis or recombinant manipulation, or be synthetically produced. Naturally occurring allelic variants can be identified with the use of well-known molecular biology techniques, such as, for example, with polymerase chain reaction (PCR) and hybridization techniques. Methods for such manipulations are generally known in the art. In addition, variants of the 6Ckine proteins can be prepared by mutagenesis or recombinant manipulations. Methods for mutagenesis and nucleotide sequence alterations are well known in the art. See, for example, Kunkel, 1985, Proc. Natl. Acad. Sci. USA 82:488-492: Kunkel et al., 1987, Methods in Enzymol. 154:367-382; US Patent No. 4,873,192: Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al., 1978, Atlas of Protein Sequence and Structure (Natl. Biomed. Res. Found., Washington, D.C.), herein incorporated by reference.

Thus, the polypeptides useful in the methods of the invention encompass both naturally occurring proteins as well as variations and modified forms thereof. Such variants will continue to possess the desired 6Ckine activity discussed above. Since only mouse but not human 6Ckine binds to the CXCR3 receptor, presumably mediating the angiostatic activity, the polypeptides useful in the method may include variants of human 6Ckine which are able to be biologically active through the CXCR3 receptor. Obviously, the mutations that will be made in the DNA encoding the variant must not place the sequence out of reading frame and preferably will not create complementary regions that could produce secondary mRNA structure. See, EP Patent Application Publication No. 75,444.

By "6Ckine polypeptide fragment" is intended a portion of the amino acid sequence of 6Ckine. As used herein, 6Ckine polypeptide fragments will retain at least 30% of the dendritic cell chemoattractant activity, anti-tumor activity, or the angiostatic activity of the full length 6Ckine polypeptide. In addition, for the purposes of the invention, a 6Ckine fragment will comprise at least 10 contiguous amino acid residues of the full length 6Ckine polypeptide. Thus, 6Ckine polypeptide fragments may range from at least 10 contiguous amino acid residues of full length 6Ckine, about 15, about 20, about 25, about 30, about 40, about 50, about 60, about 70, about 80, about 90, about 100, and up to 110-11 contiguous amino acid residues of the full length polypeptide.

Variant 6Ckine proteins and full length polypeptide fragments useful in the methods of the present invention must possess 6Ckine biological activity. Specifically, they must possess the desired biological activity of the native protein, that is, the dendritic cell-chemoattractant activity, angiostatic activity or anti-tumor activity as described herein. In addition, variants of human 6Ckine may possess angiostatic activity similar to the native mouse 6Ckine protein. For the purposes of the invention, a "6Ckine variant" will exhibit at least 30% of a dendritic cell-chemoattractant activity, tumor inhibitory activity or angiostatic activity of the full length 6Ckine polypeptide. More typical, variants exhibit more than 60% of at least one of these activities; even more typically, variants exhibit more than 80% of at least one of these activities.

The deletions, insertions, and substitutions of the protein sequences useful herein are not expected to produce radical changes in the characteristics of the protein, with the exception of intended modification of the human protein to introduce angiostatic activity similar to that of the mouse protein. However, when it is difficult to predict the exact effect of the substitution, deletion, or insertion in advance of doing so, one skilled in the art will appreciate that the effect will be evaluated by routine screening assays. That is, the dendritic cell chemoattractant activity can be evaluated by standard chemotaxis assays known to those skilled in the art. See, for example, Rubbert et al., 1998, J Immunol 160: 3933 and Nagira et al., 1997, J Biol Chem 272:19518; herein incorporated by reference. Similarly, angiostatic activity can be measured in egg chorioallantoic membrane or rodent cornea models of angiogenesis. See, for example, Soto et al., 1998, Proc Natl Acad Sci USA 95: 8205, the contents of which are incorporated herein by reference. Anti-tumor activity may be determined using implantation of tumor cells in non-human mammalian subjects, as described in Examples 2 through 5.

Amino acids which are involved in binding of 6Ckine to CCR7 are essential for chemotactic activity to 6Ckine to dendritic cells. Such amino acids can be identified by methods known in the art. Such methods include alanine-scanning mutagenesis, molecular evolution (Crameri et al., 1996, Nat. Biotechnol. 14:315-319; Crameri et al., 1998, Nature 15:288-291; Patten et al., 1997, Curr. Opin. Biotechnol. 8:724-733; Stemmer, W. P.,1994, Proc. Natl. Acad. Sci USA 91:10747-51; Stemmer, W.P., 1994, Nature 370:389-391), or site-directed mutagenesis. See, Cunningham et al., 1989, Science 244:1081. Resulting mutants can be tested for biological activity. Sites critical for binding can be determined by structural analysis such as crystallization, photoaffinity labeling, or nuclear magnetic resonance. See, deVos et al., 1992, Science 255:306 and Smith et al., 1992, J. Mol. Biol. 224:899.

A "targeting moiety" as referred to herein is a moiety which recognizes or targets a tumor-associated antigen or a structure specifically expressed by non-cancerous components of the tumor, such as the tumor vasculature. Examples of targeting moieties according to the invention include but are not limited to peptides, proteins, small molecules, vectors, antibodies or antibody fragments which recognize or target tumor-associated antigens or structures specifically expressed by non-cancerous components of a tumor. In preferred embodiments, the targeting moiety is a peptide, a protein, a small molecule, a vector such as a viral vector, an antibody or an antibody fragment. In more preferred embodiments, the targeting moiety is an antibody or antibody fragment. In most preferred embodiments, the targeting vector is a ScFv fragment.

The targeting moiety can be specific for an antigen expressed by tumor cells, as it has been described in humans, for example, for the folate receptor (Melani et al., 1998, Cancer Res. 58: 4146-4154), Her2/neu receptor, Epidermal Growth Factor Receptor and CA125 tumor antigen (Glennie et al., 2000, Immunol. Today 21: 403-410). Several other tumor antigens can be used as targets and are either preferentially expressed, uniquely expressed, over-expressed or expressed under a mutated form by the malignant cells of the tumor (Boon et al., 1997, Curr. Opin. Immunol. 9: 681-683). These may include: Melan-A, tyrosinase, p97, β-HCG, GalNAc, MAGE-1, MAGE-2, MAGE-3, MAGE-4, MAGE-12, MART-1, MUC1, MUC2, MUC3, MUC4, MUC18, CEA, DDC, melanoma antigen gp75, HKer 8, high molecular weight melanoma antigen, K19, Tyr1 and Tyr2, members of the pMel 17 gene family, c-Met, PSA, PSM, α-fetoprotein, thyroperoxidase, gp100, insulin-like growth factor receptor (IGF-R), telomerase and p53. This list is not intended to be exhaustive, but merely exemplary of the types of antigen which may be used in the practice of the invention. Alternatively, the targeting moiety can be specific for an antigen preferentially expressed by a component of the tumor different from the malignant cells, and in particular tumor blood vessels. The family of alpha v integrins, the VEGF receptor and the proteoglycan NG2 are examples of such tumor blood vessel-associated antigens (Pasqualini et al., 1997, Nat. Biotechnol. 15: 542-546).

Antibodies and antibody fragments useful as targeting moieties according to the invention can be selected according to methods known in the art. Specifically, antibodies can be derived after immunizing mammals with the selected target antigen, thus generating monoclonal antibodies. Alternatively, antibody fragments can be derived from phage display libraries, among which they are selected based on their specificity for the target antigen. In both cases, a portion of the antibody not involved in antigen recognition can be modified so that it would be less immunogenic in the patient than the original antibody fragment. Such process has been termed "antibody humanization". For generation of such antibody constructs see, for example, Hudson et al., 1999, "Recombinant Antibody Constructs in Cancer Therapy," Curr. Opin. Immunol. 11:548-557.

Targeting constructs in which the targeting moiety is an antibody or antibody fragment may be modified in a number of ways to improve their specificity and efficacy. For example, affinity of antibodies can be improved by constructing multivalent fragments or by affinity maturation. (Hudson et al., 1999, Curr. Opin. Immunol. 11:548-557).

In another aspect of the invention, the targeting moiety can be a peptide of at least 10 amino-acid that specifically binds to a tumor-associated antigen, expressed by tumor cells or the tumor vasculature (Burg et al., 1999, Cancer Res. 59: 2869-2874; Pasqualini et al., 1996, Nature 380: 364-366, Arap et al., 1998, Science 279: 377-380). The targeting moiety can also be a protein or a natural or synthetic small molecule that binds to the tumor-associated antigen, for example the natural ligand of the antigen or variants of this ligand, or a small molecule which binds this antigen.

Peptides and proteins that bind with specificity to a known tumor-associated antigen or other tumor-associated structure can be selected according to the skill in the art. For example, a targeting moiety which is a protein may be selected from the known ligand for a given tumor-associated target, variants of the known ligand, or other proteins which interact with the tumor-associated target in a binding partner-binding partner fashion, or in a natural physiologically relevant protein-protein interaction, either covalent or noncovalent. Drug screening using the tumor-associated target can be performed to identify proteins which have binding affinity to the tumor associated target.

Targeting moieties which are small molecules may also be identified by known screening procedures. In particular, it is well known in the art how to screen for small molecules which specifically bind a given target, for example tumor-associated molecules such as receptors. See, e.g., Meetings on High Throughput Screening, International Business Communications, Southborough, MA 01772-1749.

In another aspect of the invention, the targeting moiety can be a vector which delivers 6Ckine to the tumor. For example, viral vectors such as adenovirus (see Example IV), retrovirus, poxvirus, lentivirus, or herpes virus or a DNA vector may be used. The specific targeting of viral vectors to tumors can be achieved by using the natural tropism of certain viruses for certain organs or tissues. For example, vectors derived from the genus herpesviridae have been shown to have preferential infection of neuronal cells (see, e.g., U.S. Patent No. 5,328,688). Alternatively, conditionally replicating viral vectors can be used to achieve selective expression of 6Ckine in particular cell types while avoiding untoward broad spectrum infection. Examples of conditionally replicating vectors are described in WO 00/22137 published April 20, 2000; Pennisi, E., 1996 Science 274: 342-343; Russell, S.J., 1994 Eur. J. of Cancer 30A(8): 1165-1171. Additionally, the viral genome may be modified to include inducible promoters which achieve replication or expression of the transgene only under certain conditions. Examples of inducible promoters are known in the scientific literature (See, e.g. Yoshida et al., 1997 Biochem. Biophys. Res. Comm. 230:426-430; Lida, et al., 1996, J. Virol. 70(9): 6054-6059; Hwang, et al., 1997 J. Virol 71(9): 7128-7131; Lee, et al., 1997, Mol. Cell. Biol. 17(9): 5097-5105; and Dreher et al., 1997, J. Biol. Chem 272(46); 29364-29371. The 6Ckine transgene may also be under control of a tissue specific promoter region allowing expression of the transgene only in particular cell types.

Cell type specificity or cell type targeting may also be achieved in vectors derived from viruses having characteristically broad infectivities by the modification of the viral envelope proteins. For example, cell targeting has been achieved with adenovirus vectors by selective modification of the viral genome knob and fiber coding sequences to achieve expression of modified knob and fiber domains having specific interaction with unique cell surface receptors. Examples of such modifications are described in Wickham et al., 1997 J. Virol 71(11): 8221-8229 (incorporation of RGD peptides into adenoviral fiber proteins); Arnberg et al., 1997 Virology 227: 239-244 (modification of adenoviral fiber genes to achieve tropism to the eye and genital tract); Harris and Lemoine, 1996, TIG 12(10): 400-405; Stevenson et al., 1997, J. Virol. 71(6): 4782-4790; Michael et al., 1995, Gene Therapy 2: 660-668 (incorporation of gastrin releasing peptide fragment into adenovirus fiber protein); and Ohno et al., 1997, Nature Biotechnology 15: 763-767 (incorporation of Protein A-IgG binding domain into Sindbis virus).

Other methods of cell specific targeting have been achieved by the conjugation of antibodies, antibody fragments, peptides or ligands to the viral surface (see, e.g. Haisma et. al., 2000, Cancer Gene Ther. 7: 901-904; Martin et al., 1999, J. Virol 73: 6923-6929;

Michael, et al., 1993, J. Biol. Chem 268: 6866-6869, Watkins et al., 1997, Gene Therapy 4: 1004-1012; Douglas et al,. 1996, Nature Biotechnology 14: 1574-1578; Nilson et al., 1996, Gene Therapy 3: 280-286 (conjugation of EGF to retroviral proteins)).

Specificity of DNA vectors can be improved by inclusion of a DNA-binding cationic tail within the vector (Chen et al., 1998, Cancer Gene Ther. 5: 357-364) or by inclusion of the DNA vector within a lipid structure such as liposomes that preferentially extravasate from blood into tumors.

The specificity for tumors of the different targeting constructs described above can be analyzed by specific binding in vitro to tumor cells or blood vessels in comparison with non relevant tumors or healthy tissues (Melani et al., 1998, Cancer Res. 58: 4146-4154). It can be further analyzed by measuring the distribution in blood, tumor and other organs of a 6Ckine construct radiolabelled with for example $^{111}$In, $^{125}$I or $^{99}$Tec (Melani et al., 1998, Cancer Res. 58: 4146-4154; Adams et al., 1998, Brit. J. Cancer 77: 1405-1412; Pasqualini et al., 1996, Nature 380: 364-366).

Many other modifications of the targeting constructs methods of the invention can be made to improve their specificity and efficacy. For example, modification of the targeting construct such as by pegylation or inclusion in liposomes can increase their half-life in the blood and therefore their efficacy (Chapman et al., 1999, Nature Biotechnol. 17: 780-783). Different combinations or modifications of the targeting constructs of the invention may be used to provide optimal function with different ethnic groups, sex and geographic distributions.

The mode of delivery of the various types of targeting constructs of the invention may be by injection, including intravenous, intratumoral, intradermal, intramuscular and subcutaneous, or topical, such as an ointment or a patch.

The targeting constructs of the invention may be administered alone or combined with substances allowing for their slow release at delivering site (depot).

Both primary and metastatic cancer can be treated in accordance with the invention. Types of cancers which can be treated include but are not limited to melanoma, breast, pancreatic, colon, lung, glioma, hepatocellular, endometrial, gastric, intestinal, renal, prostate, thyroid, ovarian, testicular, liver, head and neck, colorectal, esophagus, stomach, eye, bladder, glioblastoma, and metastatic carcinomas. The term "carcinoma" refers to malignancies of epithelial or endocrine tissues including respiratory system carcinomas, gastrointestinal system carcinomas, genitourinary system carcinomas, prostatic carcinomas, endocrine system carcinomas, and melanomas. Metastatic, as this term is used herein, is defined as the spread of tumor to a site distant to regional lymph nodes.

In another embodiment of the invention, the targeting construct is targeted to a disease-associated antigen different from the antigens associated with cancer. This includes antigens derived from organisms known to cause diseases in man or animal such as bacteria, viruses, parasites (e.g., Leishmania) and fungi. This also includes antigens specifically expressed during the course of auto-immune diseases or inflammatory states and allergens expressed by plants or animals. The invention also relates to the use of such a targeting construct for the manufacture of a medicament for treating a disease state in a mammal.

A moiety designed to activate, induce or stimulate maturity of the DC may be advantageously administered. Examples of such agents are TNF-α, IFN-α, RANK-L or agonists of RANK, CD40-L or agonists of CD40 and agonists of the toll-like receptor family of molecules such as CpG oligonucleotides (Hemmi et al., 2000, Nature 408: 740-745). Such activating agents can provide maturation signals which i) increase the expression of CCR7, a receptor for 6Ckine, ii) drive the migration of DC from tissues toward lymphoid organs through the draining lymph, iii) activate DC to secrete molecules which enhance immune responses - in particular the anti-tumor response - such as IL-12 and IFN-α (Banchereau et al. 1998, Nature 392: 245-252). In the embodiment of the invention where the targeting construct is delivered by the means of a plasmid vector, these nucleic acid sequences may be part of the vector.

GM-CSF and IL-4 can advantageously be administered in combination with the 6Ckine fusion construct. GM-CSF and IL-4 may be administered for purposes of increasing the number of circulating DC which might then be locally recruited locally in the tumor be the subsequent injection of 6Ckine fusion protein(s). This protocol would imply a systemic pre-treatment for a least five to seven days with GM-CSF and IL-4. An alternative would be to favor by local administration of GM-CSF and IL-4 the local differentiation of DC-precursors (monocytes) into DC which could then pick up the antigen delivered at the same site. Other proteins such as FIT3-L, or a fusion protein comprising FLT-3L and G-CSF or FLT-3L and GM-CSF could also be used in place of GM-CSF and IL-4.

In addition, other chemokines or combinations of multiple chemokines may be advantageously administered in combination with the 6Ckine targeting constructs of the invention. Chemokines which have been shown to have beneficial effects include MIP-1α, MIP-3α, MIP-5, RANTES, SDF-1, Teck, DC tactin-β, MDC, MCP-1, MCP-2, MCP-3, and MCP-4 (see, e.g., Sozzani et al., 1995, J. Immunol. 155:3292-3295; Sozzani et al., 1997, J. Immunol. 159: 1993-2000; Xu et al., 1996, J. Leukoc. Biol. 60; 365-371; MacPherson et al., 1995, J. immunol. 154: 1317-1322; Roake et al., 1995, J. Exp. Med 181:2237-2247 and European Patent Application EP 0 974 357 A1 filed July 16, 1998 and published January 26, 2000).

Generally, targeting constructs and/or activating agent(s) and/or cytokine(s) are administered as pharmaceutical compositions comprising an effective amount of chemokine(s) and/or antigen(s) and/or activating agent(s) and/or cytokine(s) in a pharmaceutical carrier. These reagents can be combined for therapeutic use with additional active or inert ingredients, e.g., in conventional pharmaceutically acceptable carriers or diluents, e.g., immunogenic adjuvants, along with physiologically innocuous stabilizers and excipients. A pharmaceutical carrier can be any compatible, non-toxic substance suitable for delivering the compositions of the invention to a patient.

The quantities of reagents necessary for effective therapy will depend upon many different factors, including means of administration, target site, physiological state of the patient, and other medicants administered. Thus, treatment dosages should be titrated to optimize safety and efficacy. Animal testing of effective doses for treatment of particular cancers will provide further predictive indication of human dosage. Various considerations are described, e.g., in Gilman et al. (eds.) (1990) Goodman and Gilman's: The Pharmacological Bases of Therapeutics, 8th Ed., Pergamon Press; and Remington's Pharmaceutical Sciences, 17th ed. (1990), Mack Publishing Co., Easton, PA. Methods for administration are discussed therein and below, e.g., for intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, New Jersey. Slow release formulations, or a slow release apparatus may be used for continuous administration.

Dosage ranges for targeting constructs and/or activating agent(s) would ordinarily be expected to be in amounts lower than 1 mM concentrations, typically less than about 10 μM concentrations, usually less than about 100 nM, preferably less than about 10 pM (picomolar), and most preferably less than about 1 fM (femtomolar), with an appropriate carrier. Generally, treatment is initiated with smaller dosages which are less than the optimum dose of the compound. Thereafter, the dosage is increased by small increments until the optimum effect under the circumstance is reached. Determination of the proper dosage and administration regime for a particular situation is within the skill of the art.

Dosage of targeting construct in which the targeting moiety is a vector will largely depend on the efficacy of the particular vector employed and the condition of the patient, as well as the body weight or surface area of the patient to be treated. The size of the dose also will be determined by the existence, nature, and extent of any adverse side-effects that accompany the administration of a particular vector, or transduced cell type in a particular patient. In determining the effective amount of the vector to be administered in the treatment, the physician evaluates circulating plasma levels of the vector, vector toxicities, progression of the disease, and the production of anti-vector antibodies. The typical dose for a nucleic acid is highly dependent on route of administration and gene delivery system. Depending on delivery method the dosage can easily range from about 1 µg to 100 mg or more. In general, the dose equivalent of a naked nucleic acid from a vector is from about 1 µg to 100 µg for a typical 70 kilogram patient, and doses of vectors which include a viral particle are calculated to yield an equivalent amount of therapeutic nucleic acid.

The preferred biologically active dose of GM-CSF and IL-4 in the practice of the claimed invention is that dosing combination which will induce maximum increase in the number of circulating $CD14^+$/$CD13^+$ precursor cells; the expression of antigen presenting molecules on the surface of DC precursors and mature DC; antigen presenting activity to T cells; and/or stimulation of antigen-dependent T cell response consistent with mature-DC function. In the practice of the invention the amount of IL-4 to be used for subcutaneously administration typically ranges from about 0.05 to about 8.0 µg/kg/day, preferably 0.25 - 6.0 µg/kg/day, most preferably 0.50 - 4.0 µg/kg/day. The amount of GM-CSF is to be used for subcutaneous administration typically ranges from about 0.25 µg/kg/day to about 10.0 µg/kg/day, preferably from about 1.0 - 8.0 µg/kg/day, most preferably 2.5 - 5.0 µg/kg/day. An effective amount for a particular patient can be established by measuring a significant change in one or more of the parameters indicated above.

## EXAMPLES

The invention can be illustrated by way of the following non-limiting examples.

## EXAMPLE I

### Response of human dendritic cells to h6Ckine chemokine

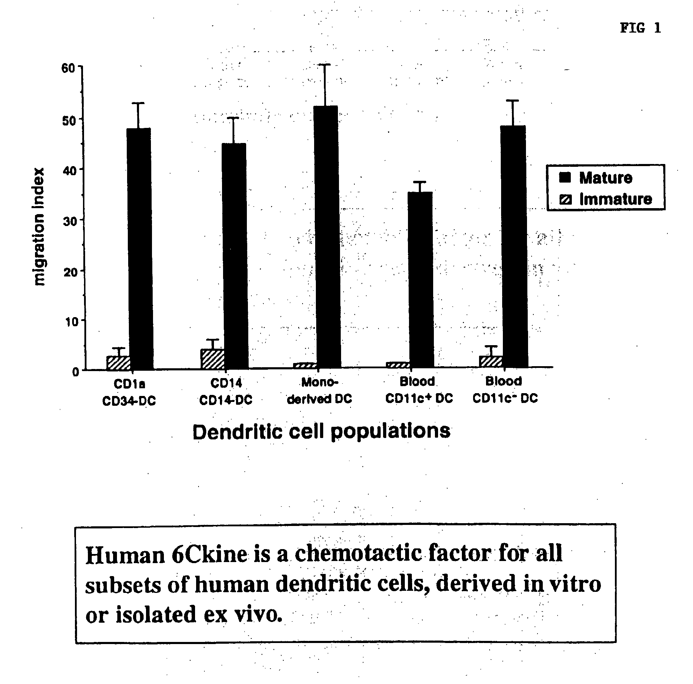

In this example, the inventors have shown that human 6Ckine ("h6Ckine") is a chemotactic factor for all known subsets of dendritic cells in man, in vitro. In particular, h6Ckine is active on human blood dendritic cells following a short incubation period with GM-CSF, IL-3 and CD40L (Fig. 1).

Different human DC populations including CD1a+ Langerhans cell, CD14+ interstitial DC, monocyte-derived DC, circulating blood CD11c+ DC, monocytes, and circulating blood CD11c- plasmacytoid DC were studied in migration assay, in response to human 6Ckine before and after maturation. CD34-derived DC precursors were isolated by Facs-sorting according to CD1a and CD14 expression after 6 days of cultures in presence of GM-CSF+TNF and SCF. Cells were cultured until day 12 in GM-CSF alone (immature) or GM-CSF+CD40-L (mature) for the last two days. Monocyte-derived DC were generated by culturing monocytes in presence of GM-CSF+IL-4 for 5 days and activated (mature) or not (immature) with CD40-L for the last 2 days. Human circulating blood CD11c+ DC and CD11c- plasmacytoid DC were enriched by magnetic bead depletion, and facs-sorted using triple staining into lineage markers FITC negative, HLA-DR tricolor positive, and CD11c PE positive and negative. CD11c+ DC and CD11c-plasmacytoid DC were cultured for three hours in presence of GM-CSF+IL-3 with (mature) or without (immature) CD40-L. Migration assays were carried out during 1 to 3 hours using 5 or 8 µm pore size Transwell (6.5mm diameter, COSTAR, Cambridge, MA), and revealed by facs analysis. All populations respond to 6Ckine but only after CD40-L activation. Similar chemotactic responses to 6Ckine were obtained after activation with TNFα and LPS (not shown).

### Hematopoietic factors, reagents and cell lines.

rhGM-CSF (specific activity: $2.10^6$ U/mg, Schering-Plough Research Institute, Kenilworth, NJ), rhTNFα (specific activity: $2 \times 10^7$ U/mg, Genzyme, Boston, MA) rhSCF (specific activity: $4 \times 10^5$ U/mg, R&D Systems, Abington, UK), and rhIL-4 (specific activity: $2.10^7$ U/mg, Schering-Plough Research Institute, Kenilworth, NJ) were used at the optimal concentration of 100 ng/ml, 2.5 ng/ml, 25 ng/ml, and 50U/ml, respectively. Recombinant human 6Ckine/CCL21, was from R&D Systems and was used at optimal concentration (1 µg/ml). The murine CD40 ligand transfected cell line (CD40-L L cells) was used as stimulator of DC maturation.

### Enrichment for $CD11c^+$ and CD11c- DC from peripheral blood.

Circulating blood $CD11c^+$ and CD11c- DC were prepared from peripheral blood as previously described [Grouard et al., 1996, Nature 384(6607) 364-367]. Briefly, peripheral blood mononuclear cells were isolated by Ficoll-Hypaque and lineage positive cells were removed using antibodies anti-CD3 (OKT3), anti-CD19 (4G7), anti-CD14 (MOP9), anti-CD56 (NKH1, Coulter), anti-CD16 (ION16, Immunotech), anti-CD35 (CR1, Immunotech), and anti-glycophorin A (JC159, DAKO) and magnetic beads (anti-mouse Ig-coated Dynabeads, Dynal). All the procedures of depletion and staining were performed in presence of 0.5 mM EDTA. The enriched population contained between 15 to 25% $CD11^+$ Dcand 10-30% CD11C- DC, identified on the expression of HLA-DR (tricolor, Becton Dickinson), CD11c (PE, Becton Dickinson) and lack of lineage markers (FITC) CD1a (Ortho Diagnostic System, Raritan, NJ); CD14, CD15, CD57, CD16, CD20, CD3 (Becton Dickinson).

### Generation of DC from cord blood $CD34^+$ HPC

$CD34^+$ cells were isolated from cord blood mononuclear fractions through positive selection as described (Caux et al., 1996, J. Exp. Med. 184:695-706;

Caux et. al., 1990, Blood 75:2292-2298), were cultured in the presence of SCF, GM-CSF and TNFα and 2.5% AB[+] human serum as described (Caux et al., 1996, J. Exp. Med. 184:695-706), in endotoxin-free complete medium consisting of RPMI 1640 (Gibco, Grand Island, NY) supplemented with 10% (v/v) heat-inactivated fetal bovine serum (FBS) (Flow Laboratories, Irvine, UK), 10 mM Hepes, 2 mM L-glutamine, 100 µg/ml gentamicin (Schering-Plough, Levalois, France). Optimal conditions were maintained by splitting these cultures at day 4 in the same conditions, and at day 6 and 10 in complete medium supplemented with GM-CSF alone without AB[+] human serum until day 12.

## Isolation of peripheral blood monocytes

Monocytes were purified by immunomagnetic depletion (Dynabeads, Dynal Oslo, Norway) after preparation of PBMC followed by a 52% Percoll gradient. The depletion was performed with anti-CD3 (OKT3), anti-CD19 (4G7), anti-CD8 (OKT8), anti-CD56 (NKH1, Coulter) and anti-CD16 (ION16, Immunotech) monoclonal antibodies. Monocyte-derived dendritic cells were produced by culturing purified monocytes for 6-7 days in the presence of GM-CSF and IL-4 (Sallusto et al., 1994, J. Exp. Med. 179:1109-1118).

## Chemotaxis assay in transwells.

Migration assays were carried out using Transwell (6.5 mm diameter, COSTAR, Cambridge, MA) with $5 \times 10^5$ cells/well. CD34[+] HPC-derived DC precursors were incubated for 1 hour in 5 µm spore size inserts and migrating cells were analyzed by double staining either for CD1a and CD14. Monocytes and monocyte-derived DC were incubated for 2 hour in 5 µm pore size inserts and migration was revealed by CD14 and/or CD1a staining. Enriched blood DC populations were incubated for 3 hours in 3 µm pore size inserts and the migration was revealed by triple staining for CD11c[±] / HLA-DR[+] / lineage.

## EXAMPLE II

### Chemokine m6Ckine gene transfer in tumor models

In this example, the inventors have shown that:

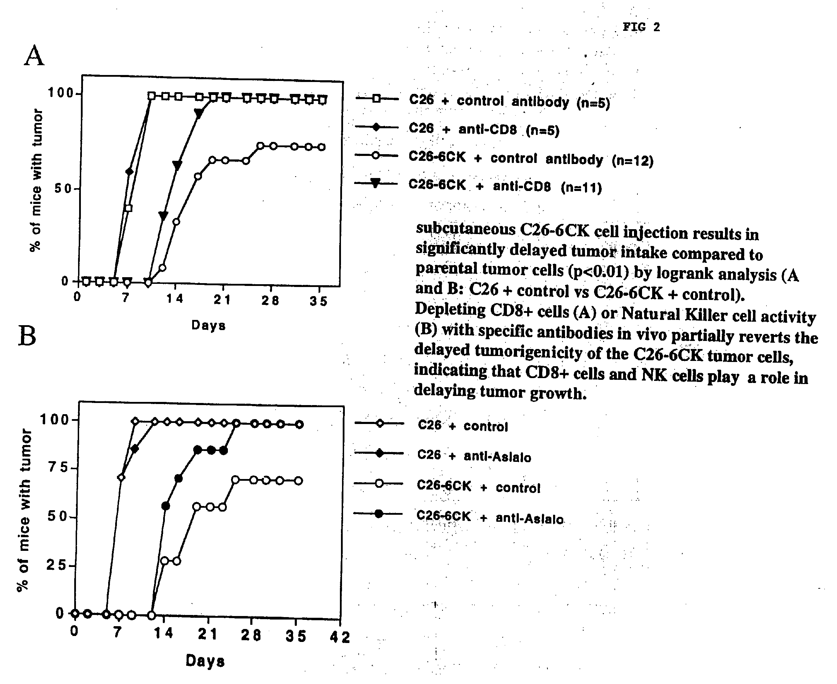

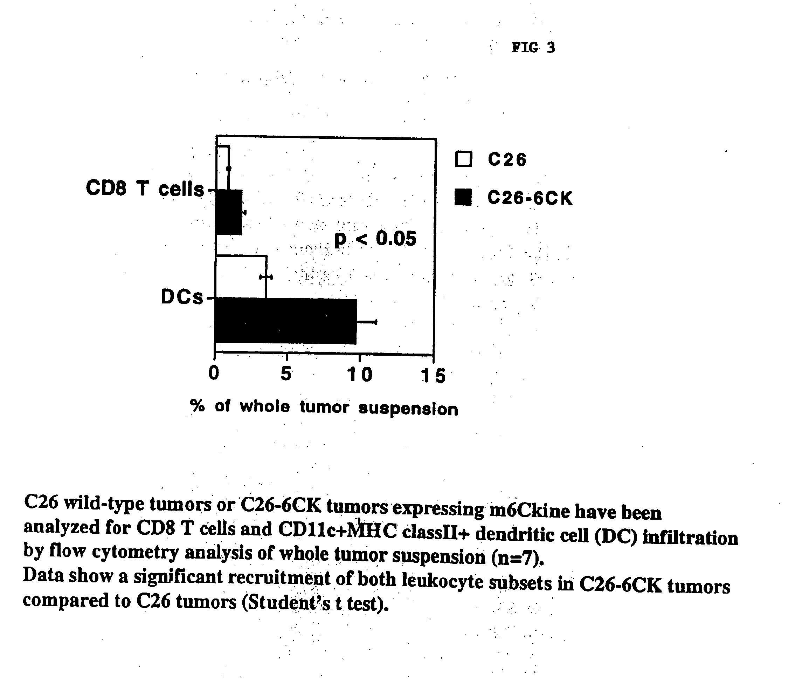

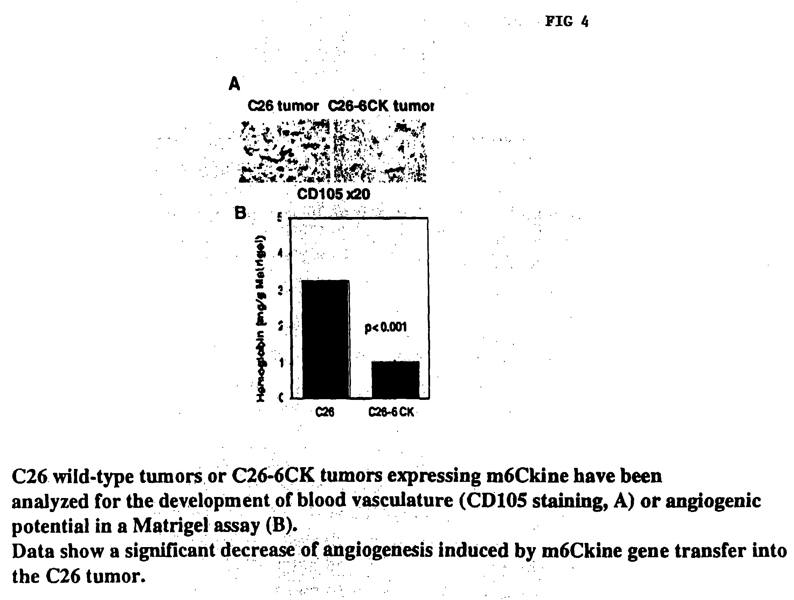

- C26 colon carcinoma tumor cells engineered to express m6Ckine are less tumorigenic and that this effect depends on CD8+ cells and Natural Killer cell activity, in vivo. (Fig.2.)
- C26 tumors expressing m6Ckine are significantly infiltrated by dendritic cells and CD8+T cells compared with parental tumors. (Fig.3.)
- C26 colon carcinoma tumor cells engineered to express m6Ckine are less angiogenic than the parental C26 tumor. (Fig.4.)

6- to 10-week-old female BALB/c (H-2[d]) mice were purchased from Charles River (Iffa-Credo, L'Arbresle, France) and maintained in our facilities under standard conditions. Procedures involving animals and their care were conducted in conformity with EEC (European Economic Community) Council Directive 86/609, OJL 358,1, December 12, 1987.

All tumor cell cultures were performed in DMEM (Gibco-BRL, Life Technologies, Paisley Park, Scotland) supplemented with 10% FCS (Gibco-BRL), 1 mM hepes (Gibco-BRL), Gentallin (Sobering-Plough, Union, NJ), $2 \times 10^{-5}$ M beta-2 mercaptoethanol (Sigma, St Louis, MO). All cell cultures were performed at 37°C in a humidified incubator with 5% $CO_2$. The cDNA encoding mouse 6Ckine (m6Ckine) was cloned into the pcDNA3 vector (InVitrogen, Carlsbad, CA) which contains a CMV promoter. C26 colon carcinoma tumor cells (kindly provided by Mario P. Colombo, Instituto Nazionale per lo Studio e la Cura dei Tumori, Milano, Italy) were transfected with this construction using the Fugene reagent (Roche Molecular Diagnostics, Mannheim, Germany) according to the manufacturer's instructions. Single C26 clones expressing m6Ckine/SLC mRNA (C26-6CK) were obtained after neomycin (Sigma) selection at 800 µg/ml. C26 or C26-6CK tumor cells were injected s.c. in the right flank in 100 µl DMEM and tumor growth was monitored by palpation three times a week. For antibody depletion, 0.5 mg of anti-CD8 (clone 2.43), rat control (GL113) purified antibodies or 200 µl rabbit anti-asialo GM1 serum (Wako Pure Chemicals, Osaka, Japan) were injected i.p. in 200 µl PBS one day before tumor inoculation, then 0.2 mg of antibodies or 100 µl anti-asialo GM1 serum were injected after three days and once a week during the course of the experiment. Figure 1 shows that subcutaneous C26-6CK cell injection results in significantly delayed tumor intake compared to parental tumor cells (p<0.01) by logrank analysis (A and B: C26 + control vs C26-6CK + control). Depleting CD8+ cells (A) or Natural Killer cell activity (B) with specific antibodies in vivo partially reverts the delayed tumorigenicity of the C26-6CK tumor cells, indicating that CD8+ cells and NK cells play a role in delaying tumor growth.

Tumors were surgically removed when reaching an approximate size of 1 cm. The tumor mass was minced into small fragments and incubated in collagenase A (Roche Molecular Biochemicals) solution for 30 min at 37°C under agitation. The suspension was then washed several times in DMEM. Staining of cell suspensions was performed in PBS + 5% FCS. Prior to incubation with FITC-, biotin- or PE-labeled specific antibodies, Fc receptors were blocked using Fc-Block™ CD16/CD32 antibody (PharMingen, San Diego, CA). The various antibodies (all from PharMingen) used in this study were CD8β (53-5.8), CD11c (HL3), anti-MHC class II I-A[d]/I-E[d] (269), CD3 (145-2C11). Biotinylated antibodies were revealed with PE-streptavidin (Becton Dickinson). Phenotypic parameters were acquired on a FacScan (Becton Dickinson, Mountain View, CA) and analyzed using the CellQuest software (Becton Dickinson). In Figure 2, C26 wild-type tumors or C26-6CK tumors expressing m6Ckine have been analyzed for CD8 T cells and CD11c+MHC classII+ dendritic cell (DC) infiltration by flow cytometry analysis of whole tumor suspension (n=7). Data show a significant recruitment of both leukocyte subsets in C26-6CK tumors compared to C26 tumors (Student's t test). These results suggest that m6Ckine gene transfer into tumors promote both the recruitment of dendritic cells, which are essential cells to initiate immune responses, including anti-tumor responses, as well as CD8 T cells, which are effector cells of the adaptive immune response.

In some experiments, tumors were removed from animals and embedded in OCT compound (Miles laboratory, Elkhart, IN) before being snap frozen in liquid nitrogen and store at -80°C until immunohistochemistry procedures. Five-micrometer cryostat sections applied onto glass slides were fixed in acetone and incubated with 1% $H_2O_2$ for 10 min at room temperature. Slides were then incubated with the biotin-block™ and avidin-block™ reagents (both from Vector, Burlingame, CA). All incubations were followed by three 2 min-washes in PBS (Gibco-BRL). Slides were then pre-incubated for 30 min with a 1/10 dilution of serum from the same species of the secondary antibody (Dako, Glostrup, Denmark). Slides were then incubated sequentially with 5 µg/ml of purified CD105 (clone MJ7/18, PharMingen, San Diego, CA), biotinylated secondary antibody (rabbit anti-rat from Vector), streptavidin-alkaline (ABC kit from Vector). Enzyme reaction was developed with the corresponding Vector substrate. Angiogenesis assays were carried out by determining the hemoglobin content of Matrigel (Becton Dickinson, Bedford, MA) pellets containing developing tumors cells in vivo. BALB/c_mice were injected with 0.5 ml Matrigel mixed with $2 \times 10^5$ C26 or C26-6CK cells s.c. in the abdominal midline. After nine days, Matrigel pellets were removed, the surrounding connective tissue was dissected away and pellets were liquefied in MatriSperse solution v/v (Becton Dickinson) for 90 min at 4°C. Hemoglobin content was determined by the Drabkin method (reagents from Sigma). Figure 4A shows that C26-6CK tumors are less vascularized than the parental C26 tumor. Figure 4B show that C26-6CK tumor cells are less angiogenic than C26 cells in a Matrigel assay. Overall, these results indicate that gene transfer of m6Ckine chemokine into tumor has angiostatic effect on the tumor vasculature.

## EXAMPLE III

### Local delivery of the chemokine 6Ckine into tumors in vivo

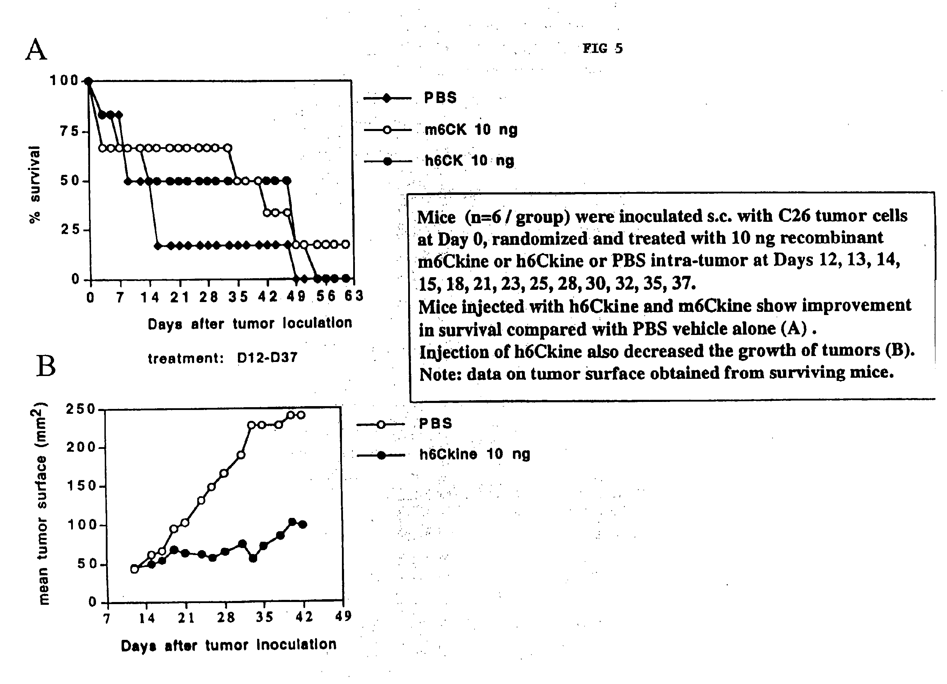

In this example, the inventors have shown that injection of recombinant human or mouse 6Ckine protein into pre-existing C26 tumors increases survival of tumor-bearing mice. Injection of h6Ckine slows tumor growth. (Fig.5).

6- to 10-week-old female BALB/c (H-2d) mice were purchased from Charles River (Iffa-Credo, L'Arbresle, France) and maintained in our facilities under standard conditions. Procedures involving animals and their care were conducted in conformity with EEC (European Economic Community) Council Directive 86/609, OJL 358,1, December 12, 1987.

All tumor cell cultures were performed in DMEM (Gibco-BRL, Life Technologies, Paisley Park, Scotland) supplemented with 10% FCS (Gibco-BRL), 1 mM hepes (Gibco-BRL), Gentallin (Schering-Plough, Union, NJ), $2 \times 10^{-5}$ M beta-2 mercaptoethanol (Sigma, St Louis, MO). All cell cultures were performed at 37°C in a humidified incubator with 5% CO2. C26 parental cells and C26 cells stably expressing the human folate receptor (C26-FR cells) as well as the J558L hybridoma were provided by Mario P. Colombo (Milano, Italy).

C26 cells were injected s.c. in the right flank in 100 µl DMEM and tumor growth was monitored by palpation three times a week. In some experiments, tumor volume was monitored using a calliper and calculated as: tumor volume = small diameter² x large diameter x 0.4. For treatment with recombinant chemokines, mice were injected intra-tumorally with 10 ng >97% pure recombinant human or mouse 6Ckine (R&D Systems, Minneapolis, MN) under 50 µl PBS. Figure 5 shows that mice injected with h6Ckine or m6Ckine show improvement in survival compared with PBS vehicle alone (A). Injection of h6Ckine also decreased the growth of tumors (B). These data show that intra-tumor delivery of recombinant 6Ckine chemokines has anti-tumor effect.

## EXAMPLE IV

### rAd/6Ckine mitigation of metastatic tumors

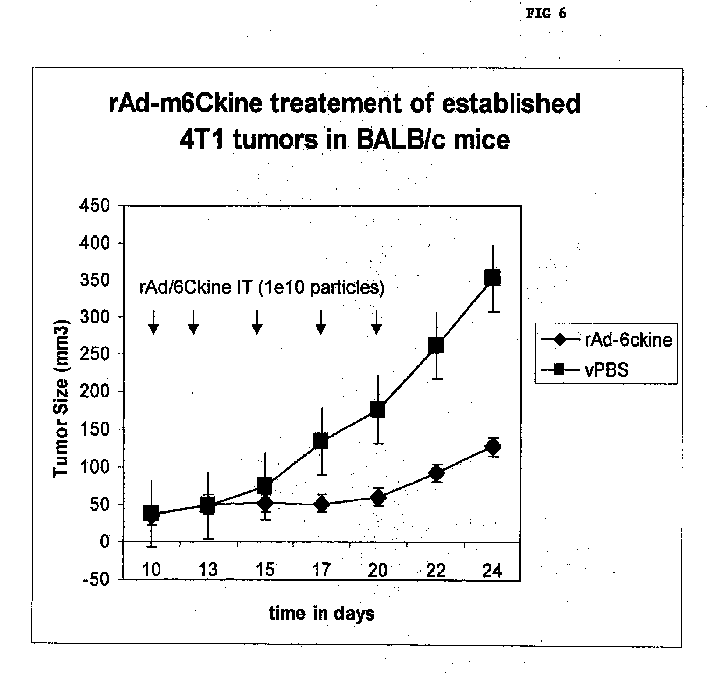

This example shows that adenoviral delivery of mouse 6Ckine inhibits tumor growth and spontaneous metastasis in established tumors in vivo (see Fig. 6).

*Construction and Purification of Recombinant Adenovirus Carrying 6CKine cDNA*

The mouse 6Ckine cDNA plasmid pT7T3D was obtained from DNAX (from Devora Rossi in the laboratory of Albert Zlotnick). Recombinant adenovirus vector carrying mouse 6Ckine cDNA was constructed by performing homologous recombination in E. coli (C. Chartier ED, M. Gantzer, A. Dieterle, A. Pavirani and M. Mehtali. Efficient Generation of Recombinant Adenovirus Vectors by Homologous Recombination in Escherichia coli. Journal of Virology 1996; 70(7):4805-10). The 6Ckine cDNA insert was excised and XbaI and BamHi sites were introduced by PCR on the 5' and 3' ends respectively (Primers: STP341-Xba6c; TAT AAT ATC TAG ATC TCA CCT ACA GCT CTG GTC TCA TC and STP342-Bam6c; TAA TAT GGA TCC AAT TGA AGT TCG TGG GGG ATC TCC). The resulting XbaI/BamHI insert containing the full length 6Ckine cDNA was ligated into XbaI/BamHI cut pTRACN"B" transfer vector (a modified shuttle vector that contains homologous sequences contained within B-backbone plasmid vector pTG4215, modified from the Ad-backbone vector received from Transgene to contain the same Ad-backbone as SCH58500; modified from vector described in Journal of Virology 1996, 70:4805). The resulting transfer vector of pTRACN "B" containing the murine 6Ckine cDNA transgene sequence was designated pTE-m6CK. The transfer vector pTE-m6CK was co-transformed with pTG4215 into E. coli (BJ5183) for recombination. The resulting recombinant viral DNA pACN-m6Ckine was then purified and verified by restriction enzyme digestion and transgene sequence analysis. The verified DNA pACN-m6Ckine DNA was linearized with Pac I and used to transfect 293 cells for generation of recombinant adenovirus, murine 6Ckine cDNA (CMCB). Viral particle (PN) concentration was determined using anion-exchange HPLC and $A_{260}$ nm measurement in 0.1% SDS (w/v). Viral construct infectivity was confirmed using a flow-cytometry based adenovirus infection assay and expressed as cellular infectious units (C.I.U.).

Female mice (BALB/c ByJ; Jackson Laboratories) were injected by subcutaneous route with $3 \times 10^5$ 4T1-p53 mammary tumor cells (syngeneic) in a volume of 0.2 ml (medium) into the left flank of animals. Animals received an intratumoral injection when the tumor grew to a size of 50-100 mm³ of 100 µl of CMCB (1e10 PN/injection) in VPBS. Mice received 3 injections per week (Monday, Wednesday, Friday) for two weeks. The tumors were measured three times weekly using a caliper (length, width, depth), the tumor volume was calculated according to formula:

$$V = 4/3 \ r^3$$

where r=(W(mm) + L(mm)+D (mm)) divided by 6 Animals were sacrificed if tumors exceed 1000 mm³.

Three mice from each group were sacrificed, starting at the time when the tumors reached 50mm³ (typically day 10), and the tumors and lungs were resected for tissue processing for the biochemical analyses described below and to assess the presence of metastases by gross and histological means.

As shown in Fig. 6, adenoviral delivery of 6Ckine inhibits tumor growth and spontaneous metastasis in established tumors by augmenting immunity and suppressing angiogenesis.

## EXAMPLE V

**Targeted Delivery of 6Ckine into tumors in vivo**

In this example, the inventors have shown that:

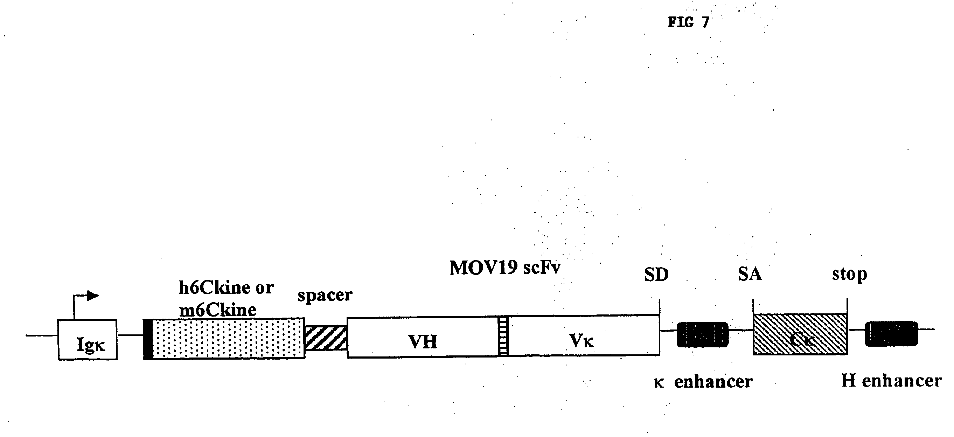

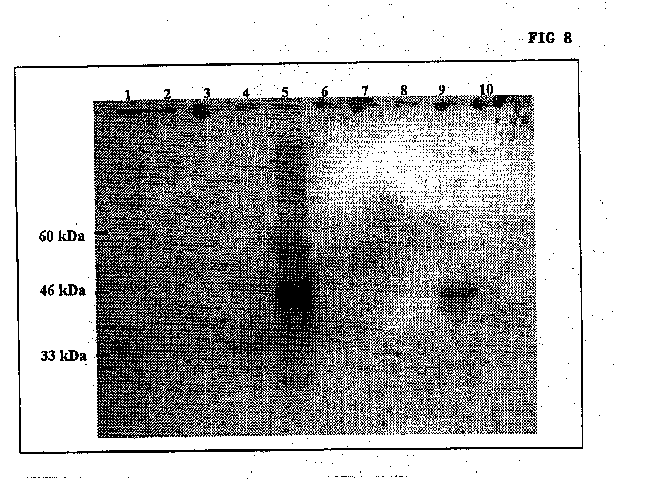

- in a targeted delivery approach, injection of hybridomas secreting a targeting construct consisting of a single chain antibody that recognizes a tumor antigen (folate receptor, FR) fused to either m6Ckine or h6Ckine specifically reduces the growth of tumors that express FR (Fig. 7).
- injection of a recombinant targeting construct consisting of a single chain antibody that recognizes a tumor antigen (folate receptor, FR) fused to h6Ckine specifically reduces the growth of tumors that express FR (Fig. 8).

*Construction and expression of h6Ckine/MOV19 ScFv and m6Ckine/MOV19 ScFv.*

The full open reading frames of m6Ckine and h6Ckine were cloned into the pcDNA3 vector (InVitrogen). Then, the human IL-2 cDNA contained in the pHIL-2/MOVSD vector (Melani et al., 1998, Cancer Res. 58: 4146-4154) obtained from Cecilia Melani and Mario Colombo, Milano, Italy was substituted by the m6Ckine or h6Ckine sequences using standard subcloning procedures. The resulting pm6CK/MOVSD and ph6CK/MOVSD vectors encode, under the immunoglobulin κ promoter, the m6Ckine or h6Ckine chemokine fused to a 13-amino acid spacer known to facilitate protein folding, then to the VH and Vκ portions of the MOV19 antibody directed against the human α-folate receptor (FR); the vectors also encode the constant portion of murine κ light chain (Cκ) and the immunoglobulin H and immunoglobulin κ enhancers (Fig. 7). A splicing consensus sequence ensures, following transcription, the fusion between Vκ and Cκ (Fig. 7). The pm6CK/MOVSD and ph6CK/MOVSD vectors were sequenced and transfected into J558L myeloma cells by protoplast fusion, and stable transfectants were selected with G418. The supernatants of resistant cells were screened by ELISA for m6Ckine or h6Ckine using specific polyclonal antibodies (R&D Systems), and the best-producing colonies subcloned to obtain high-expressing clones.

*Purification of h6Ckine/MOV19 ScFv and m6Ckine/ MOV19 ScFv fusion proteins.*

The rat anti-mouse Cκ mAb 187.1 (ATCC number HB58) was purified from high-density cultures (Integra Biosciences AG, Wallisellen, Switzerland) of the producing hybridoma grown in PFHM II protein-free hybridoma medium (Gibco BRL) by HPLC, then coupled to CNBr-activated Sepharose 4B (Pharmacia Biotech, Uppsala, Sweden) according to the manufacturer's instructions. The supernatants of J558L cells producing h6Ckine/MOV19 ScFv or m6Ckine/MOV19 ScFv grown at high density in PFHM II were dialyzed against 0.1 M Tris-HCl (pH 8.0), filtered and loaded on the Cκ affinity column. The fusion proteins were eluted with 50 mM glycin acid buffer (pH 2.5), equilibrated with 1 M Tris-base and dialyzed extensively in 3.4 nM PBS-EDTA.

The molecular size and immunoreactivity of the purified fusion proteins were tested by Western blotting. Purified proteins were subjected to 10% SDS-PAGE in nonreducing conditions and electroblotted onto Immobilon-P membranes (Millipore, Bedford, MA). After overnight saturation with PBS / 5% BSA at 4°C, filters were hybridized with 1 µg/ml horseradish peroxidase - conjugated goat anti-mouse Cκ antibody (Southern Biotechnology Associates, Birmingham, AL) in PBS / 1% BSA / 0.05% Tween 20 for one hour at room temperature. After extensive washing in PBS / 1% BSA / 0.05% Tween 20, the filters were incubated with DAB substrate solution (Pierce, Rockford, IL). The Western blotting revealed a predominant band at the expected size (46 kDa) for both h6Ckine/MOV19 ScFv (Fig 8 lane 5) and m6Ckine/MOV19 ScFv (Fig 8 lane 9) purified proteins.

Some minor bands appeared with the h6Ckine/MOV19 ScFv fusion protein, likely due to protein overload when compared to the m6Ckine MOV19 ScFv fusion protein. The specific binding of h6Ckine/ MOV19 ScFv and m6Ckine/MOV19 ScFv fusion proteins to FR, either from J558L supernatant or after affinity column purification, was checked on C26 and C26-FR cells by flow cytometry, using biotinylated anti-Cκ (Southern Biotechnology), anti-m6Ckine or anti-h6Ckine antibodies (both from R&D Systems) as secondary reagents. Both fusion proteins bind to C26-FR but not to C26 cells (data not shown). The chemotactic activity of h6Ckine/MOV19 ScFv and m6Ckine/MOV19 ScFv fusion proteins was assessed using a transwell migration assay of mouse dendritic cells. Bone marrow-derived mouse dendritic cells were obtained as previously described (Vicari et al., 2000, J. Immunol. 165: 1992-2000). Chemotaxis assays were performed in 5 µm-pore transwells (Costar, Cambridge, MA) for 2 hours at 37°C. Briefly, 600 µl of serial dilutions of h6Ckine/ MOV19 ScFv and m6Ckine/MOV19 ScFv supernatants or recombinant h6Ckine or m6Ckine in RPMI 1640 + 2% FCS were placed in the lower chamber and 2 x 105 dendritic cells in 100 µl of RPMI 1640 + 2% FCS were placed in the upper chamber. Migrated cells were collected, stained for CD11c and CD86 expression and analyzed by FACS. Results showed that h6Ckine/MOV19 ScFv and m6Ckine/MOV19 ScFv fusion proteins induced a specific chemotaxis of CD11c+CD86+ dendritic cells similar to that obtained with the recombinant chemokines, on a molar basis (data not shown).

## In vivo treatment with 6Ckine/MOV19 ScFv

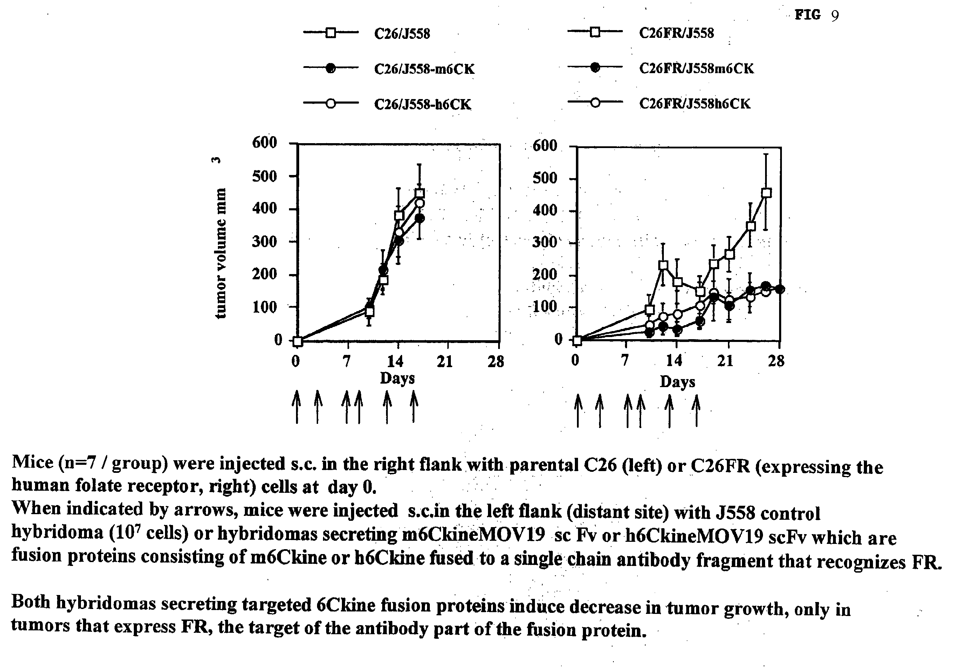

For treatment with J558 hybridoma producing 6Ckine/MOV19 constructs, mice were challenged s.c. with C26 or C26-FR tumor cells on the right flank as previously indicated, and subsequently injected s.c. in the left flank with 1 x 10[6] parental or modified to express 6Ckine/MOV19 constructs J558 cells, under a 100 µl volume of RPMI 1640 (Fig. 9). Both hybridomas secreting targeted h6Ckine or m6Ckine fusion proteins induced a significant decrease in tumor growth, only in tumors that express FR, the target of the antibody part of the fusion protein (Fig. 9).

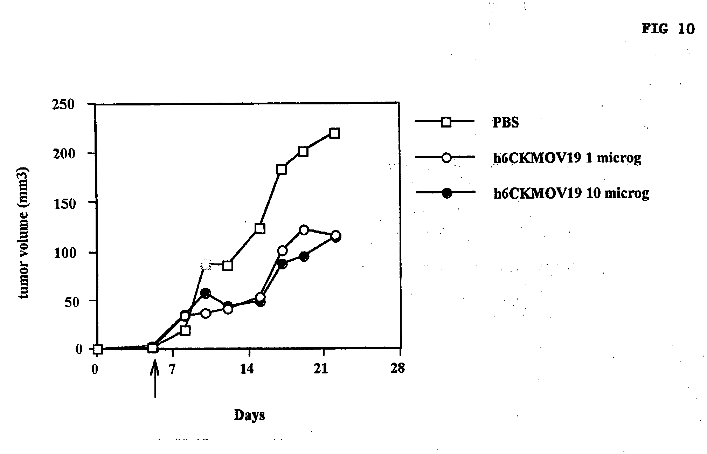

For treatment with recombinant 6Ckine/ MOV19 constructs, groups of 7 mice bearing C26-FR tumors were injected i.v. under general anesthesia with 1 µg or 10 µg of purified recombinant fusion protein (as analyzed in Fig. 8) under 200 µl of PBS, for 7 times every two days starting at day 6 (Fig. 10). Control mice were injected with PBS alone. As shown if Fig. 10, injection of targeted h6Ckine or m6Ckine fusion proteins induced a significant decrease in C26-FR tumor growth at two different doses.

Data from Fig.9 and Fig.10 indicate that targeted delivery of the chemokine 6Ckine to a tumor has anti-tumor effects.

These results indicate that targeting constructs comprising 6Ckine and an antibody fragment which recognizes a tumor-associated antigen would be a useful cancer treatment.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

## Claims

1. A targeting construct comprising 6Ckine or a biologically active fragment or variant thereof and a targeting moiety.

2. The targeting construct of claim 1 wherein the targeting moiety is a peptide of at least 10 amino acids.

3. The targeting construct of claim 1 wherein the targeting moiety is a protein.

4. The targeting construct of claim 1 wherein the targeting moiety is a small molecule.

5. The targeting construct of claim 1 wherein the targeting moiety is a vector.

6. The targeting construct of claim 5 wherein the targeting moiety is a viral vector.

7. The targeting construct of claim 1 wherein the targeting moiety is an antibody or an antibody fragment.

8. The targeting construct of claim 7 wherein the targeting moiety is an antibody fragment which recognizes the folate receptor.

9. The targeting construct of claim 1, wherein said targeting moiety recognizes a tumor associated antigen selected from the group consisting of the folate receptor, Her2/neu receptor, Epidermal Growth Factor Receptor, CA125 tumor antigen, Melan-A, tyrosinase, p97, β-HCG, GalNAc, MAGE-1, MAGE-2, MAGE-3, MAGE-4, MAGE-12, MART-1, MUC1, MUC2, MUC3, MUC4, MUC18, CEA, DDC, melanoma antigen gp75, HKer 8, high molecular weight melanoma antigen, K19, Tyr1 and Tyr2, members of the pMel 17 gene family, c-Met, PSA, PSM, α-fetoprotein, thryoperoxidase, gp100, insulin-like growth factor receptor (IGF-R), telomerase and p53.

10. The targeting construct of claim 1 wherein said targeting moiety recognizes an antigen associated with the tumor stroma, such as the tumor vasculature.

11. The targeting construct of claim 10, wherein said antigen is selected from the group consisting of alpha v integrins, the VEGF receptor and the proteoglycan NG2.

12. The targeting construct of claim 1, wherein the targeting moiety recognizes a disease-associated antigen selected from the group consisting of:

    a) an antigen derived from bacteria;
    b) an antigen derived from a virus;
    c) an antigen derived from a parasite;
    d) an antigen derived from a fungus;
    e) an antigen specifically expressed during the course of an auto-immune disease or inflammatory state; and
    f) an allergen expressed by plants or animals.

13. A targeting construct comprising 6Ckine or a biologically active fragment or variant thereof and a targeting moiety comprising an antibody fragment which recognizes the folate receptor.

14. A plasmid comprising the targeting construct of claim 1.

15. The plasmid of claim 14 further comprising a promoter sequence particularly suited for dendritic cells.

16. A targeting construct according to any one of claims 1 to 13 for use in therapy.

17. Use of a targeting construct comprising 6Ckine or a biologically active fragment or variant thereof and a targeting moiety for the manufacture of a medicament for treating cancer in a mammal.

18. Use of a targeting construct comprising 6Ckine or a biologically active fragment or variant thereof and a targeting moiety for the manufacture of a medicament for treating a disease state in a mammal, wherein the targeting moiety recognizes a disease-associated antigen selected from the group consisting of:

    a) an antigen derived from bacteria;
    b) an antigen derived from a virus;
    c) an antigen derived from a parasite;
    d) an antigen derived from a fungus;
    e) an antigen specifically expressed during the course of an auto-immune disease or inflammatory state; and
    f) an allergen expressed by plants or animals.

19. Use according to claim 17 or 18, wherein said targeting moiety is a peptide of at least 10 amino acids.

20. Use according to claim 17 or 18, wherein the targeting moiety is a protein.

21. Use according to claim 17 or 18, wherein said targeting moiety is a small molecule.

22. Use according to claim 17 or 18, wherein said targeting moiety is a vector.

23. Use according to claim 22, wherein said vector is a viral vector.

24. Use according to claim 17 or 18, wherein said targeting moiety is an antibody or antibody fragment.

25. Use according to claim 17, wherein said targeting moiety is an antibody fragment which recognizes the folate receptor.

26. Use according to claim 17, wherein said targeting moiety recognizes a tumor associated antigen selected from the group consisting of the folate receptor, Her2/neu receptor. Epidermal Growth Factor Receptor, CA125 tumor antigen, Melan-A, tyrosinase, p97, β-HCG, GalNAc, MACE-1, MAGE-2, MAGE-3, MAGE-4, MAGE-12, MART-1, MUC1, MUC2, MUC3, MUC4, MUC18, CEA, DDC, melanoma antigen gp75, HKer 8, high molecular weight melanoma antigen, K19, Tyr1 and Tyr2, members of the pMel 17 gene family, c-Met, PSA, PSM, α-fetoprotein, thryoperoxidase, gp100, insulin-like growth factor receptor (IGF-R), telomerase and p53.

27. Use according to claim 17, wherein said targeting moiety recognizes an antigen associated with the tumor stroma, such as the tumor vasculature.

**28.** Use according to claim 27, wherein said antigen is selected from the group consisting of alpha v integrins, the VEGF receptor and the proteoglycan NG2.

**29.** Use according to claim 17 or 18, wherein said medicament further comprises a substance which allows for the slow release of said targeting construct at a delivery site.

**30.** Use according to claim 17 or 18, wherein said medicament is administered intravenously, intratumorally, intradermally, intramuscularly, subcutaneously or topically.

**31.** Use according to claim 17 or 18, wherein said medicament is administered with a combination of GM-CSF and IL-4.

**32.** Use according to claim 17 or 18, wherein said medicament further comprises a combination of GM-CSF and IL-4, for prior or concurrent administration.

**33.** Use according to claim 17 or 18, wherein said medicament is administered with FLT3-L or a fusion protein comprising FLT3-L and G-CSF or GM-CSF.

**34.** Use according to claim 17 or 18, wherein said medicament further comprises FLT3-L or a fusion protein comprising FLT3-L and G-CSF or GM-CSF, for prior or concurrent administration.

**35.** Use according to claim 17 or 18, wherein said medicament is administered with an activating agent.

**36.** Use according to claim 35, wherein said activating agent is selected from the group consisting of TNF-$\alpha$, IFN-$\alpha$, RANK-L, agonists of RANK, CD40-L, agonists of CD40 and agonists of the toll-like receptor family of molecules.

**37.** Use according to claim 17, wherein said cancer is selected from the group consisting of melanoma, breast, pancreatic, colon, lung, glioma, hepatocellular, endometrial, gastric, intestinal, renal, prostate, thyroid, ovarian, testicular, liver, head and neck, colorectal, esophagus, stomach, eye, bladder, glioblastoma, and metastatic carcinomas.

FIG 1

**Dendritic cell populations**

Human 6Ckine is a chemotactic factor for all subsets of human dendritic cells, derived in vitro or isolated ex vivo.

EP 1 256 354 A1

FIG 2

A

—□— C26 + control antibody (n=5)

—◆— C26 + anti-CD8 (n=5)

—○— C26-6CK + control antibody (n=12)

—▼— C26-6CK + anti-CD8 (n=11)

subcutaneous C26-6CK cell injection results in significantly delayed tumor intake compared to parental tumor cells (p<0.01) by logrank analysis (A and B: C26 + control vs C26-6CK + control). Depleting CD8+ cells (A) or Natural Killer cell activity (B) with specific antibodies in vivo partially reverts the delayed tumorigenicity of the C26-6CK tumor cells, indicating that CD8+ cells and NK cells play a role in delaying tumor growth.

B

—◇— C26 + control

—◆— C26 + anti-Asialo

—○— C26-6CK + control

—●— C26-6CK + anti-Asialo

FIG 3

C26 wild-type tumors or C26-6CK tumors expressing m6Ckine have been analyzed for CD8 T cells and CD11c+MHC classII+ dendritic cell (DC) infiltration by flow cytometry analysis of whole tumor suspension (n=7).
Data show a significant recruitment of both leukocyte subsets in C26-6CK tumors compared to C26 tumors (Student's t test).

FIG 4

**A**

C26 tumor  C26-6CK tumor

CD105 x20

**B**

p < 0.001

C26 wild-type tumors or C26-6CK tumors expressing m6Ckine have been
analyzed for the development of blood vasculature (CD105 staining, A) or angiogenic
potential in a Matrigel assay (B).
Data show a significant decrease of angiogenesis induced by m6Ckine gene transfer into
the C26 tumor.

EP 1 256 354 A1

A

Days after tumor loculation

treatment: D12-D37

B

Days after tumor Inoculation

FIG 5

Mice (n=6 / group) were inoculated s.c. with C26 tumor cells at Day 0, randomized and treated with 10 ng recombinant m6Ckine or h6Ckine or PBS intra-tumor at Days 12, 13, 14, 15, 18, 21, 23, 25, 28, 30, 32, 35, 37.

Mice injected with h6Ckine and m6Ckine show improvement in survival compared with PBS vehicle alone (A) .

Injection of h6Ckine also decreased the growth of tumors (B).

Note: data on tumor surface obtained from surviving mice.

FIG 6

rAd-m6Ckine treatement of established
4T1 tumors in BALB/c mice

EP 1 256 354 A1

FIG 7

FIG 8

EP 1 256 354 A1

**FIG** 9

Legend:
- —□— C26/J558
- —●— C26/J558-m6CK
- —○— C26/J558-h6CK
- —□— C26FR/J558
- —●— C26FR/J558m6CK
- —○— C26FR/J558h6CK

Mice (n=7 / group) were injected s.c. in the right flank with parental C26 (left) or C26FR (expressing the human folate receptor, right) cells at day 0.

When indicated by arrows, mice were injected s.c.in the left flank (distant site) with J558 control hybridoma ($10^7$ cells) or hybridomas secreting m6CkineMOV19 sc Fv or h6CkineMOV19 scFv which are fusion proteins consisting of m6Ckine or h6Ckine fused to a single chain antibody fragment that recognizes FR.

Both hybridomas secreting targeted 6Ckine fusion proteins induce decrease in tumor growth, only in tumors that express FR, the target of the antibody part of the fusion protein.

FIG 10

EP 1 256 354 A1

**European Patent Office**

## EUROPEAN SEARCH REPORT

Application Number

EP 01 40 1211

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 00 38706 A (CHIRON CORP ;WINTER JILL A (US); CHAN VIVIEN W F (US); KETING CHU) 6 July 2000 (2000-07-06) * page 4, line 6 - line 23 * | 1-37 | A61K47/48 C07K14/52 |
| Y | EP 0 706 799 A (MERCK PATENT GMBH) 17 April 1996 (1996-04-17) * claim 1 * | 1-37 | |
| A | WO 92 08495 A (ABBOTT BIOTECH INC) 29 May 1992 (1992-05-29) * claim 1 * | 1-37 | |

| | TECHNICAL FIELDS SEARCHED (Int.Cl.7) |
|---|---|
| | A61K C07K |

The present search report has been drawn up for all claims

| Place of search | Date of completion of the search | Examiner |
|---|---|---|
| MUNICH | 17 July 2001 | DEFFNER, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 01 40 1211

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2001

| Patent document cited in search report | | Publication date | Patent family member(s) | | Publication date |
|---|---|---|---|---|---|
| WO 0038706 | A | 06-07-2000 | AU | 2715700 A | 31-07-2000 |
| EP 0706799 | A | 17-04-1996 | AU | 702184 B | 18-02-1999 |
| | | | AU | 3053395 A | 28-03-1996 |
| | | | CA | 2158322 A | 17-03-1996 |
| | | | CN | 1123185 A | 29-05-1996 |
| | | | CZ | 9502375 A | 17-04-1996 |
| | | | HU | 75836 A | 28-05-1997 |
| | | | JP | 8099901 A | 16-04-1996 |
| | | | NO | 953650 A | 18-03-1996 |
| | | | PL | 310493 A | 18-03-1996 |
| | | | SK | 114595 A | 05-03-1997 |
| | | | US | 5824782 A | 20-10-1998 |
| | | | ZA | 9507808 A | 07-05-1996 |
| WO 9208495 | A | 29-05-1992 | AU | 660297 B | 22-06-1995 |
| | | | AU | 9059691 A | 11-06-1992 |
| | | | CA | 2095836 A,C | 10-05-1992 |
| | | | EP | 0574395 A | 22-12-1993 |
| | | | JP | 9506761 T | 08-07-1997 |
| | | | US | 5650150 A | 22-07-1997 |